# EUROPEAN PATENT APPLICATION

(11) **EP 1 518 602 A1**
(43) Date of publication of application: **30.03.2005**
(21) Application number: 04024598.7
(22) Date of filing: 18.04.2002
(51) Int. Cl.: B01J 35/04, B01J 35/10, C07C 209/36

(54) **Coated monolith substrate and catalysts comprising it as support**

(30) Priority: 20.04.2001 US 839699; 30.05.2001 US 867959; 26.10.2001 US 2250
(62) Divisional of application: 02008232.7
(71) Applicant: AIR PRODUCTS AND CHEMICALS, INC., Allentown, PA 18195-1501 (US)
(72) Inventor: Nordquist, Andrew Francis, Whitehall, PA 18052 (US); Wilhelm, Frederick Carl, Zionsville, PA 18092 (US); Waller, Francis Joseph, Allentown, PA 18103-9670 (US); Machado, Reinaldo Mario, Allentown, PA 18104 (US)
(74) Representative: Schwabe - Sandmair - Marx

(57) **Abstract**

The present invention relates to monolith catalysts comprising a catalytic metal deposited onto a coated monolith substrate comprising a wash coat applied to a monolith substrate wherein the monolith catalysts have a surface area ranging from 0.1 to 25 m²/gram as measured by adsorption of N2 or Kr using the BET method. The wash coat is formed from furfuryl alcohol-containing polymer forming solution or a prepolymer containing polymerized units of furfuryl alcohol. The invention also relates to the coated monolith substrates used in such monolith catalysts. The monolith catalysts of the present invention are particularly suited toward use in hydrogenation processes which employ an immiscible mixture of an organic reactant in water.

## Description

### CROSS-REFERENCE TO RELATED APPLICATIONS

This application is a continuation-in-part of United States Serial No. 09/867,959 having a filing date of May 30, 2001, entitled, "Polymer Network/Carbon Layer on Monolith Support and Monolith Catalytic Reactor", which is a continuation-in-part of United States Serial No. 09/839,699 having a filing date of April 20, 2001, entitled "Hydrogenation With Monolith Reactor Under Conditions Of Immiscible Liquid Phases", the specifications and claims which are incorporated herein by reference and made a part of this application.

### STATEMENT REGARDING FEDERALLY SPONSORED RESEARCH OR DEVELOPMENT

The subject matter presented in this patent application was funded in part by the United States Department of Energy (DOE) under Cooperative Agreement No. DE-FC02-OOCH11018. The DOE may possess certain rights under the claims appended hereto.

### BACKGROUND OF THE INVENTION

Industrial hydrogenation reactions are often performed by using finely divided powdered slurry catalysts in stirred-tank reactors. These slurry phase reaction systems are inherently problematic in chemical process safety, operability and productivity. The finely divided, powdered catalysts are often pyrophoric and require extensive operator handling during reactor charging and filtration. By the nature of heat cycles required during start-up and shut-down, slurry systems promote co-product formation which can shorten catalyst life and lower yield of the desired product.

An option to the use of finely divided powder catalysts in stirred reactors has been the use of pelleted catalysts in fixed bed reactors. While this reactor technology does eliminate much of the handling and waste problems, a number of engineering challenges have not permitted the application of fixed bed reactor technology to hydrogenation of many organic compounds. Controlling the overall temperature rise and temperature gradients in the reaction process has been one problem.

Monolith catalytic reactors are an alternative to fixed bed reactors and provide a number of advantages over conventional fixed bed reactors. Monolith catalytic reactors exhibit a low pressure drop during operation which allows operation at higher gas and liquid velocities than achievable with fixed bed reactors. The higher velocities of gas and liquids achievable in monolith catalytic reactors promote high mass transfer and mixing and the parallel channel design of conventional monolith substrates inhibits coalescence of the gas in the liquid phase.

Research continues in developing monolith catalytic reactors to enhance catalytic activity, selectivities and catalyst life. High reaction rates can only be achieved by efficient exposure of the catalytic metal in the monolith catalytic reactor to the reactants. However, efforts to enhance exposure of the catalytic metal to the reactants are often at odds with enhancing adhesion of the metal to the monolith substrate. Embedding the catalytic metal in a coating applied to the surface of the monolith may result in greater adhesion of the catalytic metal but also reduces catalytic activity.

Hatziantoniou, et al. in "The Segmented Two-Phase Flow Monolith catalyst Reactor. An Alternative for Liquid-Phase Hydrogenations", ind. Eng. Chem. Fundam., Vol. 23, No.1, 82-88 (1984) discloses the liquid phase hydrogenation of nitrobenzoic acid (NBA) to aminobenzoic acid (ABA) in the presence of a solid palladium monolith catalyst. The monolith catalyst consisted of a number of parallel plates separated from each other by corrugated planes forming a system of parallel channels having a cross sectional area of 2 mm² per channel. The composition of the monolith comprised a mixture of glass, silica, alumina, and minor amounts of other oxides reinforced by asbestos fibers with palladium metal incorporated into the monolith in an amount of 2.5% palladium by weight. The reactor system was operated as a simulated, isothermal batch process. Feed concentrations between 50 and 100 moles /m³ were cycled through the reactor with less than 10% conversion per pass until the final conversion was between 50% and 98%.

Hatziantoniou, et al. in "Mass Transfer and Selectivity in Liquid-Phase Hydrogenation of Nitro Compounds in a Monolith catalyst Reactor with Segmented Gas-Liquid Flow", Ind. Eng. Chem. Process Des. Dev., Vol. 25, No.4, 964-970 (1986) discloses the isothermal hydrogenation of nitrobenzene and m-nitrotoluene dissolved in ethanol using a monolithic support impregnated with palladium. The authors report that the activity of the catalyst is high and therefore mass-transfer is rate determining. Hydrogenation was carried out at 590 and 980 kPa at temperatures of 73 and 103°C. Less than 10% conversion per pass was achieved. Ethanol was used as a co-solvent to maintain one homogeneous phase.

US 4,743,577 discloses metallic catalysts which are extended as thin surface layers upon a porous, sintered metal substrate for use in hydrogenation and decarbonylation reactions. In forming a monolith, a first active catalytic material such as palladium is extended as a thin metallic layer upon a surface of a second metal present in the form of porous, sintered substrate. The resulting catalyst is used for hydrogenation, deoxygenation and other chemical reactions. The monolithic metal catalyst incorporates catalytic materials such as palladium, nickel and rhodium, as well as platinum, copper, ruthenium, cobalt and mixtures. Support metals include titanium, zirconium, tungsten, chromium, nickel and alloys.

US 5,250,490 discloses a catalyst made by an electrolysis process for use in a variety of chemical reactions such as hydrogenation, deamination and amination. The catalyst is comprised of a noble metal deposited or fixed in place on a base metal, the base metal being in form of sheets, wire gauze, spiral windings and so forth. The preferred base metal is steel which has a low surface area, e.g., less than 1 square meter per gram of material. Catalytic metals which can be used to form the catalysts include platinum, rhodium, ruthenium, palladium, iridium and the like.

US 6,005,143 discloses a process for the adiabatic hydrogenation of dinitrotoluene in a monolith catalyst employing nickel and palladium as the catalytic metals. A single phase dinitrotoluene/water mixture in the absence of solvent is cycled through the monolith catalyst under plug flow conditions for producing toluenediamine.

EPO 0 233 642 discloses a process for the hydrogenation of organic compounds in the presence of a monolith-supported hydrogenation catalyst. A catalytic metal, e.g., Pd, Pt, Ni, or Cu is deposited on or in the monolith support. A variety of organic compounds are suggested as being suited for use including olefins, nitroaromatics and fatty oils.

A report by Delft University, in Elsevier Science B.V., "Preparation of Catalysts" VII, p. 175-183 (1998) discloses a carbon coated ceramic monolith in which carbon serves as a support for catalytic metals. Ceramic monolith substrates were dipped in furfuryl alcohol based polymer forming solutions and allowed to polymerize. After solidification, the polymers were carbonized in flowing argon to temperatures of 550°C followed by partial oxidation in 10% O₂ in argon at 350°C. The carbon coated monolith substrate typically had a surface area of 40-70 m²/gram.

Those skilled in the art continue to search for improved monolith catalysts which overcome problems associated with poor selectivity, low activity and unduly short catalyst life.

### BRIEF SUMMARY OF THE INVENTION

Those skilled in the catalytic arts are searching for monolith catalysts which exhibit improved adhesion of the catalytic metal to the monolith substrate and improved catalyst activity, selectivity and extended life during operation. The current state of the art teaches that improved catalytic activity of a monolith catalyst is proportional to increase in surface area of the monolith catalyst. Applicants have unexpectedly discovered that monolith catalysts having substantially improved catalytic activity can be achieved by manufacturing monolith catalysts having low surface area, defined as a surface area ranging from 0.1 to 25 m²/gram as measured by adsorption of N₂ or Kr using the BET method, as defined herein.

A first embodiment of the present invention relates to a coated monolith substrate comprising a wash coat applied to a monolith substrate wherein the coated monolith substrate has a surface area ranging from 0.1 to 25 m²/gram as measured by adsorption of N₂ or Kr using the BET method. The benefits of utilizing the claimed coated monolith substrate reside in the reduced surface area of the coated monolith substrate (i.e., the combined monolith substrate and coating) compared to conventional coated monolith substrates.

The methods employed to determine the surface area of the coated monolith substrate and the resulting monolith catalyst, referred to as the BET method, are ASTM standard methods D-4780 and D-4222. Method D-4780 utilizes krypton and is suited to measure surface areas between 10 m²/gram and about 0.1 m²/gram whereas method D-4222 utilizes nitrogen and is suited to measure surface areas greater than 10 m²/gram.

The term, monolith substrate, refers to an inorganic, ceramic or metal three-dimensional structure having a plurality of channels extending in the longitudinal direction of the structure. The monolith substrates of the present invention can be formed from any conventional monolith material including but not limited to cordierite, a carbon composite, mullite, clay, magnesia, talc, zirconia, spinel, alumina, silica, ceria, titania; tungsten, chromium, stainless steel and nickel. A preferred monolith substrate is made from cordierite. Monolith substrates may be fabricated as a honeycomb having from 100 to 800 cells per square inch.

Suitable wash coats to be deposited onto the monolith substrates to form the coated monolith substrate include a wash coat formed from a furfuryl alcohol-containing polymer forming solution or a prepolymer containing polymerized units of furfuryl alcohol. Preferably, the furfuryl alcohol-containing polymer forming solution or a prepolymer containing polymerized units of furfuryl alcohol is derived from a furfuryl alcohol/pyrrole/polyethylene glycol methyl ether solution. Other suitable wash coats include, but are not limited to silica, alumina, zirconia, titania, ceria and mixtures thereof.

A second embodiment of the present invention relates to a monolith catalyst comprising a catalytic metal deposited onto the previously recited coated monolith substrates. Suitable catalytic metals are conventional metals known to exhibit catalytic action for the reaction to be conducted. Such catalytic metals are typically selected from Groups 7, 8, 9, 10 and 11 of the Periodic Table according to the International Union of Pure and Applied Chemistry. Preferred catalytic metals include rhodium, cobalt, nickel, palladium, platinum, copper, ruthenium and rhenium. The resulting monolith catalysts has a surface area ranging from 0.1 to 25 m²/gram as measured by adsorption of N₂ or Kr using the BET method.

A third embodiment of the present invention relates to a process for producing a coated monolith substrate having a surface area ranging from 0.1 to 25 m²/gram as measured by adsorption of N₂ or Kr using the BET method suitable for use in forming a monolith catalyst comprising the steps of:
applying a wash coat comprising a furfuryl alcohol-containing polymer forming solution or a prepolymer containing polymerized units of furfuryl alcohol to a monolith substrate to form a coated monolith substrate precursor;
drying the coated monolith substrate precursor to form a dried coated monolith substrate precursor and,
heating the dried coated monolith substrate precursor to a temperature from 200° to 350°C for a time ranging from 0.1 to 3 hrs to form the coated monolith substrate having a surface area ranging from 0.1 to 25 m²/gram as measured by adsorption of N₂ or Kr using the BET method.

The coated monolith substrates and catalytic monoliths of the present invention having substantially reduced surface area compared to corresponding conventional catalysts can be readily substituted for higher surface area catalysts for use in a variety of processes.

Several advantages are achievable utilizing the embodiments of this invention including the ability:
to effect liquid phase hydrogenation of organic compounds as an immiscible phase in water and in the absence of a cosolvent;
to obtain high throughput of product through the catalytic unit even though the reaction rate may be less than that using a cosolvent;
to generate a coated monolith substrate suited for receiving a variety of catalytic metals and thereby forming a monolith catalyst having excellent activity; and
to effect hydrogenation reactions at a constant reaction rate; and, an ability to hydrogenate organic reactants under liquid phase conditions that permit ease of separation of reactants and byproduct.

### DETAILED DESCRIPTION OF THE INVENTION

Applicants have unexpectedly discovered that monolith catalysts having substantially improved catalytic activity can be achieved by manufacturing such monolith catalysts in order to achieve substantially lower surface area compared to conventional monolith catalysts, wherein surface area ranges from 0.1 to 25 m²/gram as measured by adsorption of N₂ or Kr using the BET method. The coated monolith substrates and monolith catalysts of the present invention can be readily substituted for conventional higher surface area catalysts for use in a variety of processes.

As shall be discussed in greater detail in this Specification, the coated monolith substrates and catalytic monoliths of the present invention are particularly suited for use in hydrogenation processes involving immiscible mixtures (two or more phases) of an organic reactant in water. Such immiscible mixtures can occur when water is generated during the hydrogenation reaction, or if desired, by addition of water to the organic reactant prior to or during the hydrogenation process.

A first embodiment of the present invention relates to a coated monolith substrate comprising a wash coat applied to a monolith substrate wherein the coated monolith substrate has a surface area ranging from 0.1 to 25 m²/gram as measured by adsorption of N₂ or Kr using the BET method as defined in the Brief Summary of the Invention.

The term, monolith substrate, refers to an inorganic, ceramic or metal three-dimensional structure having a plurality of channels extending in the longitudinal direction of the structure. The monolith substrates of the present invention and the monolith catalysts formed therefrom, can be formed from any conventional monolith material including cordierite, a carbon composite, mullite, clay, magnesia, talc, zirconia, spinel, alumina, silica, ceria, titania, tungsten, chromium, stainless steel and nickel. A preferred monolith substrate is made from cordierite. Preferred monolith substrates are fabricated as a honeycomb having from 100 to 800 cells per square inch.

Suitable monolith substrates include conventional honeycomb substrates formed from the enumerated materials which possess a plurality of channels, circular, square or rectangular, whereby gas and liquid can be co-currently passed through the channels under a laminar flow regime. The flow of gas and liquid in these confined channels under reaction conditions promotes "Taylor" flow with bubbles of gas, typically H₂, squeezing past the liquid. This capillary action promotes very high initial gas-liquid and liquid-solid mass transfer.

The pressure drop within the coated monolith substrates and monolith catalysts of the present invention typically range from 2 kPa/m to 200 kPa/m for combined gas/liquid superficial velocities between 0.1 to 2 meters/second for 50% gas holdup in a monolith catalyst having 400 cpi (cells per square inch). Typical dimensions for a honeycomb cell wall spacing range from 1 to 10 mm between the plates.

Typical monolith substrates may have from 100 to 800 cpi, preferably 200 to 600 cpi. Channels or cells embodied in such monolith substrates may be square, hexagonal, circular, elliptical, etc. in shape. (For purposes of convenience, it is assumed a monolith catalyst comprised of the monolith support, the enumerated coatings and a catalytic metal, has the same cpi as the monolith substrate itself).

Suitable wash coats to be deposited onto the monolith substrates include any material which is compatible with the monolith substrate. The wash coat may be formed from the same material as the monolith substrate. Optionally, the wash coat may be selected from a material compatible with the monolith substrate but not the same material as the monolith substrate including, but not limited to silica, alumina, zirconia, titania, ceria and mixtures thereof. The most preferred wash coat is formed from a furfuryl alcohol-containing polymer forming solution or a prepolymer containing polymerized units of furfuryl alcohol. Preferably, the furfuryl alcohol-containing polymer forming solution or a prepolymer containing polymerized units of furfuryl alcohol is derived from a furfuryl alcohol/pyrrole/polyethylene glycol methyl ether solution.

The wash coat can be deposited onto the monolith substrate using conventional techniques include the sol-gel method wherein a predried and evacuated monolith substrate is dipped into a suitable sol. The monolith substrate is withdrawn from the sol, drained and blown off to remove excess sol. Thereafter, the resulting coated monolith substrate can be calcined or sintered in order to obtain a coated monolith substrate having a surface area ranging from 0.1 to 25 m²/gram as measured by adsorption of N₂ or Kr using the BET method as defined in the Brief Summary of the Invention.

Suitable techniques for wash coating the monolith substrates of this invention are set forth in the book, Structured Catalysts and Reactors, edited by Andrzej Cybulski and Jacob A. Moulijn (Marcel Dekker, Inc., 1998, pp 601 -605). The amount of wash coat to be applied to the monolith substrate typically ranges from 1 to 50% of the weight of the monolith substrate, although the optimum amount may be readily determined without undue experimentation.

The optimum time and temperature for conducting the calcination/sintering step can be readily determined by one of ordinary skill in the art without undue experimentation. The practitioner may simply monitor the surface area of the coated monolith substrate during passage of time under elevated temperature.

A second embodiment of the present invention relates to a monolith catalyst comprising a catalytic metal incorporated onto the previously recited coated monolith substrates. Suitable catalytic metals are conventional metals known to exhibit catalytic activity for the reaction to be conducted. Such catalytic metals are typically selected from Groups 7, 8, 9, 10 and 11 of the Periodic Table according to the International Union of Pure and Applied Chemistry. Preferred catalytic metals include rhodium, cobalt, nickel, palladium, platinum, copper, ruthenium and rhenium. The resulting monolith catalysts has a surface area ranging from 0.1 to 25 m²/gram as measured by adsorption of N₂ or Kr using the BET method.

The catalytic metals may be deposited onto the coated monolith substrate using conventional methods known in the art. The term, deposited, refers to any conventional technique utilized to incorporate a catalytically active phase to the monolith substrate. Suitable techniques for depositing such catalytic metals to form the monolith catalysts of the present invention include conventional steps known in the art including impregnation, adsorption and ion exchange, precipitation or coprecipitation, deposition precipitation, the sol-gel method, slurry dip-coating, in situ crystallization. These methods are additional methods are set forth in the book, Structured Catalysts and Reactors, edited by Andrzej Cybulski and Jacob A. Moulijn (Marcel Dekker, Inc., 1998, pp 605 -610).

A third embodiment of the present invention relates to a process for producing a coated monolith substrate having a surface area ranging from 0.1 to 25 m²/gram as measured by adsorption of N₂ or Kr using the BET method suitable for use in forming a monolith catalyst comprising the steps of:
applying a wash coat comprising a furfuryl alcohol-containing polymer forming solution or a prepolymer containing polymerized units of furfuryl alcohol to a monolith substrate to form a coated monolith substrate precursor;
drying the coated monolith substrate precursor to form a dried coated monolith substrate precursor and,
heating the dried coated monolith substrate precursor to a temperature from 200° to 350°C for a time ranging from 0.1 to 3 hrs to form the coated monolith substrate having a surface area ranging from 0.1 to 25 m²/gram as measured by adsorption of N₂ or Kr using the BET method.

According to the first step of the process, a wash coat comprising a furfuryl alcohol-containing polymer forming solution or a prepolymer containing polymerized units of furfuryl alcohol is applied to the monolith substrate to form a coated monolith substrate precursor. Examples of polymer forming solutions suited for producing polymer network/carbon coating include furfuryl alcohol solutions and solutions of furfuryl alcohol with other additives such as pyrrole and polyethylene glycol methyl ether. The furfuryl alcohol solutions may also be based upon prepolymers containing polymerized units of furfuryl alcohol. A preferred example is a furfuryl alcohol polymer solution derived from a furfuryl alcohol/pyrrole/polyethylene glycol methyl ether solution. An example of a copolymer is one based upon furfuryl alcohol and formaldehyde.

Other examples of suitable polymer solutions include epoxy resins with amines; epoxy resins with anhydrides; saturated polyester with glycerol or other multifunctional alcohols; oil-modified alkyd saturated polyesters, unsaturated polyesters; polyamides; polyimides; phenol/formaldehyde; urea/formaldehyde; melamine/formaldehyde and others. Preferred polymer network/carbon coatings are based upon commercially available oligomers and copolymers of furfuryl alcohol as the coating solution.

The wash coat of the polymer coating solution is applied to the monolith substrate as a thin film such that the interior dimensions of the cells in the monolith support are not altered significantly in order to form a coated monolith substrate precursor. The cell dimensions of the monolith substrate and the resulting monolith catalyst are desirably maintained within the 100 to 800 cpi range.

According to the second step of the process, the coated monolith substrate precursor is dried to form a dried coated monolith substrate precursor. The drying step may be conducted by conventional methods including use of a conventional oven in air. Typical conditions include temperatures ranging from 60 to 100°C over a time period of 2 to 24 hours.

According to the third step of the process, the dried coated monolith substrate precursor is heated to a temperature from 200° to 350°C for a time ranging from 0.1 to 3 hours to form the coated monolith substrate having a surface area ranging from 0.1 to 25 m²/gram as measured by adsorption of N₂ or Kr using the BET method. This step results in partially carbonizing the polymer coating. Temperatures for partially carbonizing the polymer network/carbon coatings range from 200 to 350°C vs. 550-900°C commonly used for conventional polymer solutions known in the prior art. Because of the lower calcination temperatures used herein, network polymers having functional groups, particularly those based upon furfuryl alcohol, will retain some of their functionality and are more like the polymer than carbon. These functional groups also can be coupled through reaction chemistry to anchor homogeneous catalysts, homogeneous chiral catalysts or ligands to the polymeric surface.

The coated monolith substrates and monolith catalysts of the present invention can be utilized in a wide variety of processes including hydrogenation of organic compounds having functional groups capable of being hydrogenated. Such functional groups include nitro, anhydride, and the reaction product of a ketone or aldehyde and ammonia, aromatic amine, primary or secondary amine. Conventional reactors may be employed to conduct processes which utilize the coated monolith substrates and monolith catalysts of the present invention. Hydrogenation of organic compounds is typically effected at temperatures of 60-180°C. The hydrogenation pressure can be up to 1600 psig. The superficial liquid and gas velocities in the reactor is typically maintained to effect a desired conversion, e.g., 1% to 99% per pass. Typically, the superficial velocity through the reactor ranges between 0.1 to 2 meters per second with residence times of from 0.5 to 120 seconds.

Catalytic metals suited for the hydrogenation of water immiscible organics are impregnated directly onto the coated monolith substrate according to conventional methods. A mixture of catalytic metals may also be employed, one example being a mixture of palladium and nickel. In the case when the monolith substrate is impregnated, the catalytic metals are typically identified in units of weight percent of the monolith catalyst in which case typical catalyst metal loadings range from 0.1 to 25% by weight and preferably from 1 to 10% by weight.

Many other organic compounds are capable of undergoing a hydrogenation reaction utilizing the coated monolith substrates and monolith catalysts of this invention. Suitable nitroaromatics are nitrobenzene, nitrotoluenes, nitroxylenes, nitroanisoles and halogenated nitroaromatics where the halogen is Cl, Br, I, or F.

Anhydrides such as maleic anhydride and phthalic anhydride may be hydrogenated to γ-butyrolactone and phthalide respectively. The γ-butyrolactone can be further reduced to tetrahydrofuran.

The following examples are intended to represent various embodiments of the invention and are not intended to restrict the scope thereof.

### Preparation of Polymer Network/Carbon Coated Monolith Substrate

### General Procedure

Coating: A network polymer resin can be made from the polymerization of the appropriate monorners or oligomers. As an example furfuryl alcohol is polymerized with an acid at a controlled temperature to produce a coating solution. The acid can be inorganic (i.e. HNO₃, HCl, H₂SO₄) or organic (i.e, aromatic sulfonic). A dried monolith substrate was soaked in the desired wash coat solution for 2-4 minutes, allowed to drip dry (removal of excess coating solution from the channels). If the monolith channels had become visually blocked by the polymer wash coat solution, the channels were blown clear with air. The monolith catalyst was set in the hood for approximately 1 hr., and periodically checked to see if channels remain cleared. If channels are not clear, air was blown through the channels. The coated monolith substrate precursor was further dried at 80°C in an oven purged with N₂ purge overnight to form a dried coated monolith substrate precursor.

Calcination: The dried coated monolith substrate precursor was mounted in a tube furnace and purged with N₂ while the heat was increased to 110°C for 30 minutes. Heating was continued until the coated monolith substrate precursor surface temperature is 280°C and held at 280°C for 2 hours. The furnace was cooled to 260°C and 5% O₂/He was introduced instead of the N₂. The monolith substrate precursor was heated to 280°C and held there for 40 minutes. The carrier gas was switched back to N₂ and the heat was turned off. The resulting coated monolith substrate was removed after reaching room temperature.

Catalyst Deposition: The catalytically active metal was incorporated onto the coated monolith substrate by an incipient wetness technique, dried at 80°C in an oven overnight with N₂ purge and then calcined at a tube surface temperature of 280°C using N₂. The catalytic metal can also be pre-reduced before being used as a catalyst in a hydrogenation process. To be more specific, following calcination the amount of metal salt to dissolve or standard metal solution to be diluted was calculated based on a previously determined water uptake. In a typical example of metal impregnation, a 2" diameter 400 cpi cordierite monolith 2" in height was placed in a beaker containing approximately 80 ml of active metal solution. Additional solution was added to cover the coated monolith substrate if necessary. The coated monolith substrate was soaked for approximately 30 minutes or until no bubbles are seen. The solution was poured from the beaker, the resulting monolith catalyst was removed and excess solution from channels was cleared by a low flow of air.

The monolith catalyst was placed in an 80°C oven with N₂ purge overnight. The monolith catalyst was removed from the oven, and cooled in a desiccator. The monolith catalyst was then heated in a tube furnace at a tube surface temperature of 280°C using N₂ for 2 hours.

### Preparation of Catalyst A

### Polymer Network/Carbon Coated Monolith Substrate

Coating: Three hundred (300) ml of furfuryl alcohol, 150 ml of melted polyethylene glycol methyl ether (MW ∼750) and 90 ml of pyrrole were added to a beaker. While stirring the three component mixture, the temperature was lowered to approximately 17°C. Small increments of 70% HNO₃ (20 ml total) were added to the mixture while controlling the temperature at less than 20°C. After the addition of the acid, the mixture was stirred for 1 hour while maintaining temperature at approximately 21-23°C. The monolith substrate was placed in a beaker and sufficient polymer solution prepared above was poured to completely cover the monolith substrate. The monolith substrate was soaked until no bubbles were observed at the liquid surface.

The resulting coated monolith substrate precursor was removed from the polymer solution and drained briefly, then re-immersed in the polymer solution. The coated monolith substrate precursor was removed from the polymer solution, drained and blown with air to assure a uniform polymer coating with no blocked channels. The coated monolith substrate precursor was placed in a 80°C oven with a N₂ purge for overnight to provide the dried coated monolith substrate precursor.

Calcination/Activation: The dried coated monolith substrate precursor was placed in a quartz tube which was mounted in a vertical tube furnace. The quartz tube was purged with N₂ and heated to a tube surface temperature of 110°C at a rate of about 10°C per minute. The temperature was held at 110°C for 30 minutes. The temperature of the tube surface was increased to 280°C at 10° per minute and held at 280°C for 2 hrs. The tube surface was cooled to about 260°C. The N₂ was switched to 5% O₂ in an inert gas. The tube containing the dried coated monolith substrate precursor was heated to 280°C and held at 280°C for approximately 40 minutes. The stream of 5% oxygen in an inert gas was switched back to N₂ and a N₂ purge was maintained while cooling to room temperature to provide the coated monolith substrate.

Metal Impregnation: The amount of water absorbed by the coated monolith substrate and the metal concentration required to attain the desired metal loading were determined according to conventional methods. The coated monolith substrate was placed in a suitable container and the metal solution was poured to completely cover the coated monolith substrate. The coated monolith substrate was soaked for about 30 minutes until no bubbles were observed at the liquid surface. The monolith catalyst was removed from the container, drained and the channels were blown with air to remove any excess solution. The monolith catalyst was placed in a 80°C oven with a N₂ purge for overnight.

Monolith Catalyst Activation: The monolith catalyst was placed in a quartz tube which was mounted in a vertical tube furnace as described above under Calcination/Activation. The quartz tube was purged with N₂ for about 10 minutes. The tube surface temperature was heated to 110°C at a rate of about 10°C per minute. The temperature was held at 110°C for 30 minutes. The temperature of the tube surface was increased to 280° at 10°C per minute and held at 280°C for 2 hrs. If desirable, a reducing gas, such as 4% H₂ in N₂, may be introduced and held at 280°C for 2 hrs. The tube was purged with N₂ and cooled to ambient temperature with N₂. At ambient temperature the monolith catalyst was passivated after the reduction step in a flowing inert gas stream containing 5% O₂ for 30 minutes.

### Hydrogenation Rate Determination

A 2-liter batch autoclave was fitted with a dual-function impeller, oriented above a holder for the monolith catalyst, capable of inducing gas and pumping the gas-liquid dispersion through the monolith catalyst. For the reactions studied, the typical combined liquid volume of reagents was 1 liter. The autoclave holding the monolith catalyst was equipped with a dip tube to transfer the liquid reaction solution to a recovery cylinder. The portion of the reaction solution which was removed, was diluted and an internal standard added. Gas chromatography was used to perform a quantitative product analysis to calculate selectivity and conversion.

The raw hydrogen pressure data was corrected for compressibility. A hydrogen uptake curve was obtained as a function of reaction time. This curve was used to calculate rate data at various stages of conversion.

### Example 1

### Hydrogenation of Nitrobenzene Using a Cosolvent, Isopropanol

A series of monolith catalysts according to the present invention having varying organic coatings was used to effect the hydrogenation of nitrobenzene (NB). Hydrogenation was carried out at a concentration of 40 wt.% NB in isopropanol and the rate of hydrogenation was measured at 50% conversion. The monolith catalysts were tested in one liquid phase. Isopropyl alcohol was added as a solvent in order to make miscible the two immiscible phases of nitrobenzene and water. Reaction conditions consisted of 120°C, 200 psig H₂ at a stirring rate of 1500 rpm.

The column in Table 1 marked initial rate represents the second experimental run in the batch autoclave and the column marked final rate represents the eighth experimental run at the same set of conditions using the same monolith catalyst. The rate, at 50% conversion, is expressed in moles H₂ per m³ catalyst per second. Selectivity in mol% is determined at 100% conversion. The adsorption of N₂ or Kr using the BET method was used to measure total surface area and the units are in m²/gram. All % Pd are wt. % and based on total weight of the monolith catalyst.

**Table 1**

| Pd Monolith Catalyst in One Liquid Phase | | | | | | |
|---|---|---|---|---|---|---|
| Catalyst | Layer | Comment | Rate¹ (initial) | Rate (final) | Sel. to Aniline | Surface Area (m²/gm) |
| A | polymer network/carbon | 1.5% Pd/C/ cordierite² | 92 | 91 | 97 | <1 |
| B | polymer network/carbon | 3.1 % Pd/C/ cordierite³ | 61 | 74 | 97 | 12 |
| C | polymer network/carbon | 2% Pd/C/ cordierite^{4,5} | 47 | 20 | 97 | <1 |
| D | carbon composite | 1.7% Pd on C⁵ | 20 | 13 | 98 | 466 |
| E | carbon composite | 4.6% Pd on C^{4,5} | 36 | 23 | 93 | 372 |
| F | polymer network/carbon | 2% Pd/C/ cordierite^{4,6} | 87 | 46 | 99 | <1 |
| G (control) | No carbon | 2% Pd/ cordierite | 33 | 16 | 98 | <1 |

| | | | | | | |
|---|---|---|---|---|---|---|
| 1. Moles H₂ per m³ catalyst per second | | | | | | |
| 2. Furfuryl alcohol network polymer wash coat, low temperature calcination, metal deposition, was as in general procedure | | | | | | |
| 3. Same catalyst formulation as Catalyst A-Higher Pd loading-Calcination temperature was 550°C | | | | | | |
| 4. Metal deposition and calcination according to general procedure | | | | | | |
| 5. C, D and E are developmental monoliths from commercial vendors | | | | | | |
| 6. The wash coat was made from a phenolic resin (Varcum) | | | | | | |

Table 1 shows a general inverse trend between initial hydrogenation rate and surface area of the monolith catalyst whether a carbon composite or a polymer network/carbon layer was employed, independent of catalyst loading. Monolith catalysts having an adsorption of N₂ or Kr using the BET method of 12 or less m²/gram provided high initial and final hydrogenation reaction rates. This finding is contrary to the teachings in the scientific literature which state that a high surface area catalyst is expected to be more catalytically active than a corresponding catalyst having a lower surface area.

Except for one carbon composite based monolith catalyst obtained from a commercial vendor, all monolith catalysts based upon either a carbon composite or polymer network/carbon wash coat were more active than the control Catalyst G based on a monolith which did not have any wash coat. In addition, the monolith catalysts having wash coats made from furfuryl alcohol or a phenolic resin each have low surface area and exhibit superior initial hydrogenation rates.

In contrast, the monolith catalysts having a wash coat of the furfuryl alcohol based coating layer according to Catalysts A and B did not show a drop in hydrogenation activity after 8 experimental runs. Catalyst A which was based upon a monolith substrate and a wash coat of a polymer network/carbon coating which was calcined according to the procedures of this Specification retained some functionality vis-à-vis Catalyst B which was based upon a polymer network/carbon wash coat which was calcined at elevated temperatures according to prior art methods. Catalyst A exhibited significantly higher initial and final hydrogenation rates and at a lower catalyst metal loading than all other monolith catalysts. Except for Catalyst E (carbon composite monolith) all catalysts gave aniline selectivity greater than approximately 97 mol%.

### Example 2

### Evaluation of Monolith Catalysts For Nitrobenzene Hydrogenation Without a Cosolvent- Two-Phase

A series of monolith catalysts comprising a cordierite monolith and a polymer network/carbon wash coat were tested using neat nitrobenzene as the reactant. Conditions were similar to Example 1 except that the reaction system comprised two liquid phases. The results are shown in Table 2.

**Table 2**

| Pd Monolith Catalyst in-Two Immiscible Phases | | | |
|---|---|---|---|
| Catalyst | Layer | Rate¹ (initial) | Sel to Aniline |
| A | polymer network/carbon | 42 | 99 |
| B | polymer network /carbon | 44 | 99 |
| F | polymer network/carbon | 33 | 99 |

| | | | |
|---|---|---|---|
| 1. moles H₂ per m³ catalyst per second; 120°C; 200 psig; 1500 rpm | | | |

In each experimental run, the hydrogen uptake curve when re-plotted as the hydrogenation rate vs. time showed that the hydrogenation rate was nearly constant until toward the end of the reaction. The nearly constant hydrogenation rate was not expected since the co-product, water, is being formed during the reaction and two immiscible phases are present. As the concentration of the water increased, one of ordinary skill in the art would expect that the hydrogenation rate would decrease. In this example, Catalyst A which had half the metal loading compared to Catalyst B gave an equal hydrogenation rate.

### Example 3

### Evaluation of Monolith Catalysts Without a Cosolvent- Two-Phase

The procedure of Example 2 was repeated with the exception of the monolith catalyst utilized and the immiscible feed consisted initially of 34 wt.% nitrobenzene, 48 wt.% aniline and 18 wt.% water. The reaction temperature and pressure were 140°C and 400 psig respectively.

The hydrogenation rates for Example 3 are shown in Table 3.

**Table 3**

| Pd Monolith Catalyst in Two Immiscible Phases | | | |
|---|---|---|---|
| Catalyst | Layer | Rate¹ (initial) | Sel to Aniline |
| A | polymer network/carbon | 124 | 97 |
| D | carbon composite | 19 | 97 |
| E | carbon composite | 21 | 78 |
| G | cordierite/no carbon | 17 | 96 |

| | | | |
|---|---|---|---|
| 1. moles H₂ per m³ catalyst per second; 140°C; 400 psig; 1500 rpm | | | |

Monolith Catalyst A Monolith Catalyst D and Monolith Catalyst E gave nearly constant hydrogenation rates in two immiscible phases when the hydrogen uptake curve was re-plotted as the hydrogenation rate vs. time. There was a marked drop in aniline selectivity in the experimental run utilizing monolith Catalyst E which has a surface area outside the bounds of the claimed invention.

### Example 4

### Evaluation of Monolith Catalysts For Nitrobenzene Hydrogenation Using a Cosolvent, Isopropanol

The procedure of Example 1 was repeated with the exception of the monolith catalyst employed in the hydrogenation reaction. Monolith Catalyst J comprises a cordierite monolith having a carbon layer formed by a modified calcination procedure. The calcination procedure consisted of 650°C with a N₂ purge for 2 hours followed by 5% O₂/N₂ at 450°C for 40 minutes. The surface area by N₂ BET of the resulting monolith catalyst was 40-70 m² per gram.

Table 4 illustrates the catalytic activity of the respective monolith catalysts as a function of the extent of calcination. The Table demonstrates that the partial calcination procedure utilized to make the monolith catalysts of the present invention provide superior catalyst activity compared to monolith catalysts which undergo a complete calcination according to prior art methods. Hydrogenation was carried out at a concentration of 40 wt.% NB in isopropanol. As the surface area of the monolith catalyst increases, the hydrogenation activity decreases.

**Table 4**

| Pd Monolith Catalyst in One Liquid Phase | | | | | |
|---|---|---|---|---|---|
| Catalyst | Layer | Rate (initial)¹ | Rate (final) | Sel. to Aniline² | Surface Area (m²/gram) |
| A | polymer network/carbon | 92 | 91³ | 97 | <1 |
| B | polymer network/carbon | 61 | 74³ | 98 | 12 |
| J | carbon | 37 | 24⁴ | 99 | 40-70 |

| | | | | | |
|---|---|---|---|---|---|
| 1. Moles H₂ per m³ catalyst per second | | | | | |
| 2. Selectivity determined at final experiment | | | | | |
| 3. Final rate is the eighth experiment at the same set of conditions | | | | | |
| 4. Final rate is the seventh experiment at the same set of conditions | | | | | |

The results show that monolith catalysts A and B which possess surface areas within the bounds of the claimed invention exhibit superior catalytic activity compared to monolith Catalyst J which was prepared according to prior art methods to provide a monolith catalyst having a surface area of 40-70 m²/gram.

### Example 5

### Evaluation of Monolith Catalysts For Nitrobenzene Hydrogenation

The procedure in Example 1 was repeated and a comparison was made between one liquid phase and two liquid immiscible phases. The same molar concentration of nitrobenzene was used in the one liquid phase and two liquid immiscible phase experimental runs. Table 5 shows the rate of hydrogenation at 50% conversion for three catalysts as a function of monolith catalyst surface area.

**Table 5**

| Pd Monolith Catalyst | | | | | |
|---|---|---|---|---|---|
| Catalyst | Layer | Liquid Phases | Rate¹ | Sel. To Aniline | Surface Area (m²/gram) |
| A | polymer | 1² | 91⁴ | 97 | <1 |
| | network/carbon | 2³ | 46⁴ | 99 | |
| F | polymer | 1² | 46⁴ | 99 | <1 |
| | network/carbon | 2³ | 41⁴ | 99 | |
| J | polymer | 1² | 24⁵ | 99 | 40-70 |
| | network/carbon | 2³ | 21⁵ | 99 | |

| | | | | | |
|---|---|---|---|---|---|
| 1. moles H₂ per m³ catalyst per second; Pd/C/cordierite | | | | | |
| 2. One phase: 2.97M NB (40 wt%) in isopropanol | | | | | |
| 3. Two phases: 2.97M NB (34 wt%) in 48 wt% aniline and 18 wt% water | | | | | |
| 4. 120°C; 200 psig; 1500 rpm | | | | | |
| 5. 140°C; 200 psig; 1500 rpm | | | | | |

The monolith Catalysts, A and F, in general, have faster hydrogenation rates in either one phase or two phases when the total surface area of the monolith catalyst falls with the claimed bounds of the present invention. Monolith Catalyst A showed a difference in reaction rate depending on whether the reaction medium was one phase or two phases. Surprisingly, Catalyst F or Catalyst J had equal to or only slightly improved hydrogenation rates when going from two liquid phases to one liquid phase.

### Example 6

### Evaluation of Monolith Catalysts For Nitrobenzene Hydrogenation

The procedure in Example 1 was repeated in order to compare the activity of the monolith catalyst having a wash coat formed by polymerizing furfuryl alcohol or from a preformed co-polymer of furfuryl alcohol. The hydrogenation was carried out at a concentration of 40 wt% NB in isopropanol. Reactions conditions were 120°C, 200 psig H₂ at a stirring rate of 1500 rpm.

**Table 6**

| Pd Monolith Catalyst in One Liquid Phase | | | | | |
|---|---|---|---|---|---|
| Catalyst | Layer | Comment | Rate¹ (initial) | Sel. To Aniline | Surface Area (m²/gm) |
| A | polymer network/carbon | 2% Pd/C/ cordierite² | 92 | 97 | <1 |
| K | polymer network/carbon | 2% Pd/C/ cordierite³ | 53 | 99 | <1 |
| G (control) | no carbon | 2% Pd/ cordierite | 33 | 98 | <1 |

| | | | | | |
|---|---|---|---|---|---|
| 1. Moles H₂ per m³ catalyst per second | | | | | |
| 2. Furfuryl alcohol polymer network wash coat, low temperature calcination, metal deposition, was as in procedure | | | | | |
| 3. Co-polymer of furfuryl alcohol-formaldehyde resin and phenol sulfonic acid catalyst with pyrrole and polyethylene glycol methyl ether, low temperature calcination, metal deposition, was as in procedure | | | | | |

Monolith Catalyst K is a cordierite monolith having a polymer network/carbon coating layer formed from a wash coat solution consisting of furfuryl alcohol-formaldehyde resin, furfuryl alcohol, phenol sulfonic acid, pyrrole and polyethylene glycol methyl ether.

### Example 7

### Evaluation of Monolith Catalysts For Nitrobenzene Hydrogenation

This Example serves to directly compare catalyst activity for hydrogenation of nitrobenzene using a catalyst disclosed in Table 2 of Ind. Eng. Chem. Process Des. Dev. 1986, 25, 964-970 having a BET surface area of 80m²/gram (p. 964) to an analogous catalyst according to the present invention having a BET surface area of 19 m²/gram.

The article recited hydrogenation reaction data for nitrobenzene at 102°C, 984 kPa (146 psig) at a gas flow of 52 X 10⁻⁶ m³ per sec and a liquid flow of 16.9 X 10⁻⁶ m³ per sec. The rate of reaction (hydrogenation) is 5.0 mmol nitrobenzene/sec.kg of catalyst (pg 969). Using the density of the monolith of 1030 kg/m3 (pg 964), the new units for the rate of reaction are defined as 15.4 moles H₂ per m^{3/} catalyst per sec. The concentration of nitrobenzene in ethanol is 0.3M. The catalyst used in the article is a silica wash coat layer on a monolith substrate. The wt% Pd is 5.3% (pg 964).

The catalyst according to the present invention having a BET surface area of 19 m²/gram was prepared according to the following procedure.

A cordierite monolith substrate was dried at 120-130°C overnight. The dried monolith substrate was added to a wash coat solution made from 250 ml of Ludox AS-30 and 23g of PEG 750. The dried monolith substrate and wash coat solution were placed in a low volume container in order to cover the monolith substrate with wash coat solution. After soaking for ∼10 minutes, the article was removed, drained for ∼30 seconds to remove excess liquid, inverted and soaked an additional 10 minutes. The article was again removed, drained and the channels were cleared using compressed air.

The resulting coated monolith substrate precursor was placed in an oven overnight at 110°C. In a muffle furnace with air flow, the coated monolith substrate precursor was heated to 110°C at 8°C /minute, and held for 20 minutes. The coated monolith substrate precursor was heated to a maximum temperature of 600°C at 8°C/minute and held for 2 hrs and then cooled in air.

### Metal Impregnation:

Water capacity was determined using standard procedures known in the art. Knowing the water capacity, the Pd solution concentration was calculated to achieve a 2% wt. gain of Pd based on the wt. of the coated monolith substrate precursor. Again using a low volume container, half of the Pd solution was poured into the container and the coated monolith substrate precursor was placed into the container. The coated monolith substrate precursor was covered with the remaining Pd solution and soaked for ∼20 minutes. The article was removed from the container, drained and the channels were cleared using compressed air. The article was transferred to a drying oven and dried at 80°C overnight followed by heating in N₂ at 300°C for 2 hrs to provide the monolith catalyst.

The hydrogenation reaction was carried out at a concentration of 40 wt. % nitrobenzene in isopropanol and with a feed consisting initially of 34 wt. % nitrobenzene, 48 wt. % aniline and 18 wt. % water. The reaction conditions were 120°C, 200 psig H₂ at a stirring rate of 1500 rpm. The surface area of the resulting monolith catalyst was 19 m²/gram using the BET Method (using N₂).

**Table 7**

| **Hydrogenation of Nitrobenzene** | | |
|---|---|---|
| **Catalyst** | **Surface Area**^{**1**} | **Hydrogenation Rate** ^{**2**} |
| 5.3% Pd/silica layer³ | 80 | 15.4⁴ |
| 1.4% Pd/silica layer | 19⁵ | 50.2⁴ |

| | | |
|---|---|---|
| 1. m²/gram | | |
| 2. moles H₂ per m³ catalyst per second | | |
| 3. Ind. Eng. Chem. Process Des. Dev. 1986, 25, 964-970 | | |
| 4. 102°C, 146 psig | | |
| 5. corrected to 102°C, 146 psig using conventional calculations | | |

The data in Table 7 demonstrate that the rate of hydrogenation obtained using the monolith catalysts of the present invention is greater than the catalyst activity obtained using prior art catalysts which have surface areas substantially greater than presented in the claimed invention. This trend is also observed in Table 1 for monolith catalysts formed from a polymer network/carbon wash coat. One additional observation is that the higher hydrogenation rate has been obtained using a monolith catalyst having lower Pd loading (1.4 wt% Pd in the monolith catalyst of this invention vs 5.3 wt% Pd in monolith catalyst according to the reference). Thus, the monolith catalysts of the present invention provide superior catalyst activity at lower metal loading levels thereby reducing catalyst cost by with using less cataiytic metal.

## Claims

1. A coated monolith substrate comprising a wash coat applied to a monolith substrate wherein the coated monolith substrate has a surface area ranging from 0.1 to 25 m²/gram as measured by adsorption of N₂ or Kr using the BET method, and wherein the wash coat is formed from a furfuryl alcohol-containing polymer forming solution or a prepolymer containing polymerized units of furfuryl alcohol.

2. The monolith substrate of claim 1 wherein the furfuryl alcohol-containing polymer forming solution or a prepolymer containing polymerized units of furfuryl alcohol is derived from a furfuryl alcohol/pyrrole/polyethylene glycol methyl ether solution.

3. A monolith catalyst comprising a catalytic metal deposited onto a coated monolith substrate comprising a wash coat applied to a monolith substrate, wherein the catalyst has surface area ranging from 0.1 to 25 m²/gram as measured by adsorption of N₂ or Kr using the BET method, and wherein the wash coat is formed from a furfuyl alcohol-containing polymer forming solution or a prepolymer containing polymerized units of furfuryl alcohol.

4. The monolith catalyst of claim 3 wherein the furfuryl alcohol-containing polymer forming solution or a prepolymer containing polymerized units of furfuryl alcohol is derived from a furfuryl alcohol/pyrrole/polyethylene glycol methyl ether solution.

5. A process for producing a coated monolith substrate having a surface area ranging from 0.1 to 25 m²/gram as measured by adsorption of N₂ or Kr using the BET method suitable for use in forming a monolith catalyst comprising the steps of:
applying a wash coat comprising a furfuryl alcohol-containing polymer forming solution or a prepolymer containing polymerized units of furfuryl alcohol to a monolith substrate to form a coated monolith substrate precursor;
drying the coated monolith substrate precursor to form a dried coated monolith substrate precursor and,
heating the dried coated substrate precursor to a temperature from 200 °C to 350 °C for a time ranging from 0.1 to 3 hrs to form the coated monolith substrate having a surface area ranging from 0.1 to 25 m²/gram as measured by adsorption of N₂ or Kr using the BET method.
